(19) Europäisches Patentamt
European Patent Office
Office européen des brevets

(11) EP 1 184 362 A1

(12) EUROPEAN PATENT APPLICATION
published in accordance with Art. 158(3) EPC

(43) Date of publication:
**06.03.2002 Bulletin 2002/10**

(21) Application number: **01915840.1**

(22) Date of filing: **27.03.2001**

(51) Int Cl.[7]: **C07C 39/373**, C07C 37/18, C07C 69/73, C07C 67/343, A61K 31/055, A61K 31/216, A61K 31/192, A61K 31/235, A61K 7/00, A23G 3/30, A23G 3/00 // (C07C69/732, 59:54)

(86) International application number:
**PCT/JP01/02482**

(87) International publication number:
**WO 01/72678 (04.10.2001 Gazette 2001/40)**

(84) Designated Contracting States:
**AT BE CH CY DE DK ES FI FR GB GR IE IT LI LU MC NL PT SE TR**

(30) Priority: **28.03.2000 JP 2000088217**
**12.03.2001 JP 2001068180**

(71) Applicant: **Kabushiki Kaisha Hayashibara Seibutsu Kagaku Kenkyujo**
**Okayama-shi, Okayama 700-0907 (JP)**

(72) Inventors:
- **HORI, Hitoshi**
**Itano-gun, Tokushima 771-0207 (JP)**
- **NAKAGAWA, Y.,**
**c/o K.K.Hayashibara Seibutsu Kagaku**
**Okayama-shi,Okayama 700-0907 (JP)**

(74) Representative: **Daniels, Jeffrey Nicholas et al**
**Page White & Farrer 54 Doughty Street**
**London WC1N 2LS (GB)**

# (54) PHENOL DERIVATIVES, PROCESS FOR PREPARATION OF THE SAME AND USE THEREOF

(57) Disclosed are an efficient, safe process for producing artepillin C and derivatives thereof by an organic synthesis. The process comprises a step of reacting a phenol derivative with an acrylate or acrylic acid.

EP 1 184 362 A1

## Description

### TECHNICAL FIELD

The present invention relates to novel compounds, more particularly, to novel phenol derivatives which are useful as a material for organic synthesis of 3- [4-hydroxy-3,5-bis-(3-methyl-2-butenyl)phenyl ] -2-propenic acid and derivatives thereof, their preparations and uses.

### BACKGROUND ART

The term 3- [ 4-hydroxy-3,5-bis-(3-methyl-2-butenyl)phenyl ] -2-propenic acid and derivatives thereof (hereinafter abbreviated as "artepillin C and derivatives thereof", unless specified otherwise) is a general term for the compounds represented by Formula 5. The representative example is 3-[4-hydroxy-3,5-bis-(3-methyl-2-butenyl)phenyl]-2-propenic acid (abbreviated as "artepillin C" hereinafter) represented by Chemical Formula 2. In Formula 5, $R_1$ to $R_5$ and $R_9$ each independently represent a hydrogen atom, an aliphatic hydrocarbon group, alicyclic hydrocarbon group, or aromatic hydrocarbon group, and these hydrocarbon groups may have a substituent. $R_6$ represents a hydrogen atom or an acyl group. Artepillin C was found in natural materials such as propolis and revealed that it has many physiological activities such as antibacterial, antioxidation, anti-inflammatory, anti-tumor, apoptosis-regulation, immunoenhancement, and anti-lipidperoxidation actions. Artepillin C having such biological activities is expected for use in food products, health foods, and cosmetics to maintain/promote health conditions, and in pharmaceuticals to prevent/cure diseases. However, as mentioned below, it has not yet been established any method which can efficiently and stably provide these compounds with a relatively-high purity.

Formula 5

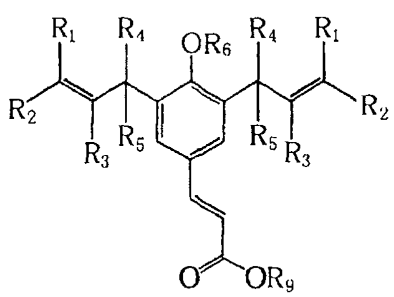

Chemical Formula 2

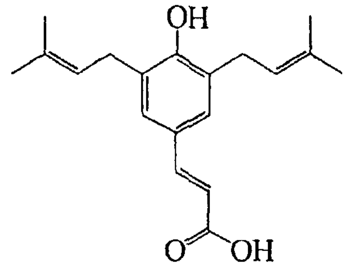

The following two methods: Hitherto known preparation methods for artepillin C are. The first method is to isolate an objective compound from natural materials such as propolis by combining purification methods such as chromatography. Japanese Patent Kokai No. 256,177/94 applied for by the same applicant as the present invention discloses that 0.18 part by weight of artepillin C crystal was obtained from 100 part by weight of propolis from Brazil. The second method is an organic synthesis as disclosed in Japanese Patent Kokai No. 163,841/85 where 0.08 mole of artepillin C crystal was obtained from one mole of p-hydroxycinnamic acid as a starting material through a step of reacting p-hydroxycinnamic acid with 1-bromo-3-methyl-2-butene.

The first method has characteristics of obtaining a desired product with relatively-high safety when applied to living bodies, but has the disadvantage that the yield of artepillin C to the material is quite low. The second method has advantageously more yields the objective compound than the first method. However, when the present inventors tried to synthesize artepillin C according to the second method, they found that the method was not necessarily useful as an industrial-scale preparation of a purified product of the compound usable in many fields, because it formed diversified by-products in the reaction step. To use as an industrial preparation, the second method should be improved its safety because of the use of dangerous highly-explosive reagents such as explosive ones are used.

### OBJECT OF THE INVENTION

In view of the foregoing, the object of the present invention is to provide a compound, which is useful as a material for organic synthesis of artepillin C and derivatives thereof, efficient and safe process for producing artepillin C and derivatives thereof, and their uses.

The present inventors started to research on the hypothesis that the above object can be solved if the objective compound were synthesized in accordance with "Heck reaction" to substitute an alkenyl group for a halogen of phenol halide by using palladium as a catalyst under relatively-moderate conditions as disclosed

by C. B. Ziegler and R. F. Heck in "Journal of Organic Chemistry", vol.43, pp.2941-2946 (1978). As the results of their repeated research, they found that phenol derivatives represented by Formula 1 were preferable as a reaction substrate to efficiently synthesize the objective compound by using the above substitution reaction. In Formula 1, $R_1$ to $R_5$ each independently represent a hydrogen atom, an aliphatic hydrocarbon group, alicyclic hydrocarbon group, or aromatic hydrocarbon group, and these hydrocarbon groups may have a substituent. $R_6$ represents a hydrogen atom or an acyl group. $X_1$ represents a substituent selected from halogens, nitro, nitroso, cyano, mercapto, sulfonic acid, and sulfonyl groups.

Formula 1

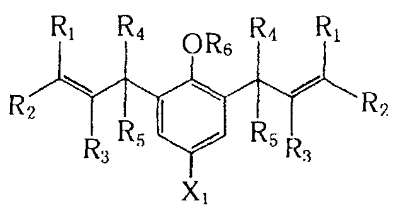

However, the phenol derivatives represented by Formula 1 have not yet been known, and the present inventors eagerly studied to establish their preparation methods. After studying materials and reaction conditions, unexpectedly, they found that the compound represented by Formula 1 can be very efficiently synthesized even under relatively-moderate conditions such as in alkaline solutions at ambient temperature, when reacting a compound represented by Formula 2, having $X_1$ which corresponds to Formula 1, with a compound represented by Formula 3 having $R_1$ to $R_5$ which correspond to Formula 1. In Formula 2, $R_7$ represents a hydrogen atom or an acyl group. In Formula 3, $X_2$ represents a halogen.

Formula 2

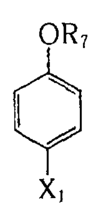

Formula 3

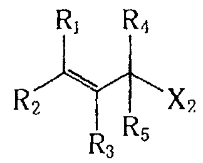

The present inventors further studied the substitution reaction of $X_1$ in the phenol derivatives represented by Formula 1 which were obtained by the above methods in accordance with Heck reaction, and found that artepillin C and derivatives thereof can be very efficiently and safely synthesized through a step of reacting the above phenol derivatives with an acrylate or acrylic acid. Thus, the present inventors accomplished this invention. The present invention was made based on the above original discovery by the present inventors.

Detailed Description of the Invention

The present invention is to provide novel phenol derivatives represented by Formula 1, which are useful as a material for organic synthesis of artepillin C and derivatives thereof, and their preparations, and to provide novel-, efficient- and safety-preparations of artepillin C and derivatives thereof using the phenol derivatives as a material, and their uses.

The phenol derivatives of the present invention mean the compounds represented by Formula 1. In Formula 1, $R_1$ to $R_5$ each independently represent a hydrogen atom, an aliphatic hydrocarbon group, alicyclic hydrocarbon group, or aromatic hydrocarbon group, and these hydrocarbon groups may have a substituent. Examples of the aliphatic hydrocarbon group are methyl, ethyl, propyl, isopropyl, butyl, isobutyl, *sec*-butyl, *tert*-butyl, pentyl, isopentyl, neopentyl, *tert*-pentyl, 1-methylpentyl, 2-methylpentyl, hexyl, isohexyl, heptyl, vinyl, aryl, and ethynyl groups. Examples of the alicyclic hydrocarbon groups are cyclopropyl, cyclobutyl, cyclopentyl, cyclohexyl, and cyclohexenyl groups. Examples of the aromatic hydrocarbon groups are phenyl, biphenylyl, *o*-tolyl, *m*-tolyl, *p*-tolyl, *o*-cumenyl, *m*-cumenyl, *p*-cumenyl, xylyl, mesityl, styryl, cinnamyl, and naphthyl groups. In Formula 1, $R_6$ represents a hydrogen atom or an acyl group. Examples of the acyl groups are acetyl, formyl, propionyl, butyryl, isobutyryl, hexanoyl, octanoyl, benzoyl, and naphthoyl groups. In Formula 1, $X_1$ represents a substituent selected from halogens, nitro, nitroso, cyano, mercapto, sulfonic acid, and sulfonyl groups. In the phenol derivatives of the present invention which are used as a material of artepillin C and derivatives thereof, it is desirable that $R_1$ to $R_5$ are a hydrogen atom or lower alkyl groups such as methyl and ethyl groups, $R_6$ is a hydrogen atom or a lower acyl group such as an acetyl group, and $X_1$ is halogen such

as iodine, bromine or chlorine. One of more desirable derivatives is a compound represented by the following Chemical Formula 1.

Chemical Formula 1

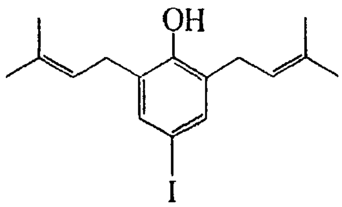

For example, the phenol derivatives of the present invention can be prepared by the process of the present invention comprising a step of reacting a compound, represented by Formula 2 having $X_1$ in Formula 1, with a compound represented by Formula 3 having $R_1$ to $R_5$ which correspond to Formula 1. In Formula 2, $R_7$ represents a hydrogen atom or an acyl group such as acetyl, formyl or propionyl group. In Formula 3, $X_2$ represents a halogen such as iodine, bromine or chlorine.

Reaction conditions in the above step are not specifically restricted, as long as form the objective phenol derivatives. For example, it is advantageous that a desirable reaction quickly proceeds, as the reaction proceeds in an appropriate polar solvent under alkaline conditions adjusted by an appropriate basic compound. Examples of the polar solvents are acetic acid, acetic anhydride, propionic anhydride, methanol, ethanol, propanol, isopropanol, cresol, benzyl alcohol, methyl cellosolve, ethyl cellosolve, acetone, acetonitrile, 1,4-dioxane, tetrahydrofuran, tetrahydropyran, formamide, N-methylformamide, N,N-dimethylformamide, N-methylpyrrolidine, dimethyl sulfoxide, water, and mixtures of at least two or more of these solvents. Examples of the basic compounds to adjust to alkaline conditions are sodium hydroxide, potassium hydroxide, sodium acetate, potassium acetate, potassium carbonate, calcium carbonate, triethylamine, N,N-dimethylaniline, piperidine, morpholine, and 1,8-diazabicyclo [5.4.0] -7-undecene. In reacting compounds represented by Formula 3 with compounds represented by Formula 2, usually, in a molar ratio of over one fold, and more desirably two fold or more, the objective phenol derivatives can be prepared in a particularly-high yield. In the above reaction step, the objective phenol derivatives may be oxidized depending on reacting conditions and result in a relatively-low yield. Accordingly, the reaction should preferably be proceeded under conditions where oxygen in a reaction vessel is replaced with inactivated gas such as argon or nitrogen gas, in the presence of an antioxidant, or under light-shielded conditions, if necessary.

Reaction mixtures containing the phenol derivatives of the present invention are obtainable through the above reaction, and optionally further through the substitution reaction with acyl, alkyl, alkenyl, and aryl groups and/or the decomposition reaction of, for example, ester bond. The optional substitution and/or decomposition reactions can be done under reaction conditions in general. The obtained reaction mixtures containing the phenol derivatives of the present invention can be used intact, and, if necessary, prior to use, further purified by the following conventional methods in general used for purifying their related compounds; dissolution, separation, extraction, decantation, filtration, concentration, thin-layer chromatography, column chromatography, high-performance liquid chromatography, distillation, sublimation, and crystallization, which can be appropriately used in combination. When used as a material for artepillin C including derivatives thereof, the above reaction mixtures should preferably be purified by silica gel chromatography etc., before use. Varying depending on reaction conditions and purification methods, the phenol derivatives can be obtained by the above processed of the present invention in a yield of usually 10% or more, preferably 20% or more, and more preferably at least about 40% in a molar ratio of the phenol derivatives to the compound represented by Formula 2 as the starting material. The phenol derivatives thus obtained can be advantageously used as a material for artepillin C as mentioned below, and, because they have activities to absorb ultraviolet rays and inhibit oxidation in themselves, can be used, for example, as ultraviolet absorbents and antioxidants in a food product, skin-whitening and skin-dressing agents in a cosmetic field, and in the form of compositions as food products and cosmetics combined with other ingredients, which are orally, percutaneously, or externally administrable to living bodies, such as starches, proteins, amino acids, fibrous materials, saccharides, fatty acids, vitamins, minerals, alcohols, and water.

When practiced the above process for producing artepillin C and derivatives thereof of the present invention, which uses the phenol derivatives of the present invention as the materials, artepillin C and derivatives thereof can be efficiently and safely produced. The artepillin C and derivatives thereof of the present invention mean compounds represented by Formula 5 as below. $R_1$ to $R_6$ in Formula 5 are the same as those defined in Formula 1. $R_9$ represents a hydrogen atom, an aliphatic hydrocarbon group, alicyclic hydrocarbon group, or aromatic hydrocarbon group. These hydrocarbon groups may have a substituent. The compounds represented by Formula 5 may have geometrical isomers, optical isomers, and rotational isomers, and these isomers are included in the artepillin C and derivatives thereof of the present invention. The compounds represented by Formula 5 may exist in salt forms, and these salts are included in the artepillin C and derivatives thereof of the present invention.

Formula 5

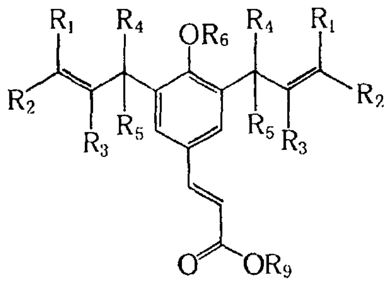

Artepillin C and derivatives thereof have many physiological activities such as antibacterial, antioxidation, anti-inflammatory, anti-tumor, apoptosis-regulatory, immunoenhancement, and anti-lipidperoxidation activities as disclosed by the same applicant as the present applicant in Japanese Patent Kokai Nos. 256,177/94, 71,528/97, and 328,425/97, by Aga et al. in "Bioscience Biotechnology and Biochemistry" , Vol. 58, pp.945-946 (1994), by Matsuda in "Foods and Food Ingredients Journal of Japan", Vol, 160, pp.64-73 (1994), by Nakano et al. in *"Mitsubachi-Kagaku",* Vol, 16, pp. 175-177 (1995), by Kimoto et al. in *"Nihon-Iji-Sinpo"*, Vol. 3726, pp. 43-48 (1995), and by Kimoto et al. in *"Dai-58-Kai-Nihon-Gangakkai-Koen-Yoshisyu*" (1999). Among the compounds represented by Formula 5, those which $R_1$ to $R_5$ and $R_9$ are a hydrogen atom or lower alkyl groups such as methyl and ethyl groups and $R_6$ is hydrogen or a lower acyl group such as an acetyl group, remarkably exert the above physiological activities. Particularly, 3- [4-hydroxy-3,5-bis-(3-methyl-2-butenyl)phenyl] -2-propenic acid represented by Formula 2 very remarkably exerts the above physiological activities and very useful in many fields such as food products, beverages, health foods, cosmetics, pharmaceuticals, household articles, agriculture, forestry, and fishery.

Chemical Formula 2

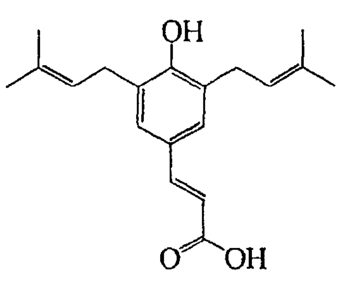

To produce artepillin C and derivatives thereof from the phenol derivatives of the present invention, it is preferable to react the phenol derivatives with acrylic acid or acrylate represented by Formula 4. In Formula 4, $R_8$ represents an aliphatic, alicyclic, or aromatic hydrocarbon group. These hydrocarbon groups may have a substituent. Examples of the aliphatic hydrocarbon group are methyl, ethyl, vinyl, allyl, ethynyl groups, etc; Examples of the alicyclic hydrocarbon groups are cyclopropyl, cyclohexyl groups, etc; Examples of the aromatic hydrocarbon groups are phenyl, tolyl, xylyl groups, etc.

Formula 4

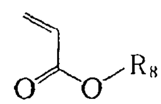

The above reaction conditions are not limited as long as the objective compound is produced. For example, the objective reaction can be proceeded in appropriate organic solvents such as toluene, acetonitrile, dimethylformamide, dimethylsulfoxide, N-methylformamide, N,N-dimethylformamide, N-methylpyrrolidone, formamide, tetrahydrofuran, tetrahydropyran, 1,4-dioxane or a mixture of at least two solvents selected from these solvents in the presence of appropriate catalysts and usually under alkaline conditions. Examples of catalysts are transition metals such as palladium, platinum, nickel, and rhodium, and ionized metals thereof. More particularly, dihydric palladium is useful in the present invention. Catalysts, in the form of a complex and/or salt may be preferably used. For example, catalysts, in the form of a complex having tri-o-toluic phosphine or triphenylphosphine as a ligand, are particularly useful in the present invention. Addition of basic materials such as triethylamine, tetrahydrofuran, pyridine, morpholine, piperidine, N,N-dimethylaniline, and 1,8-diazabicyclo [5.4.0] -7-undecene to a reaction solution enables to react under alkaline conditions. In the reaction under alkaline conditions using dihydric palladium as a catalyst, kinds and/or quantity of by-products can be remarkably suppressed. In the above reaction step, the objective artepillin C and derivatives thereof may be oxidized depending on reaction conditions, resulting in a relatively-low yield. Accordingly, the reaction can be advantageously proceeded under the conditions of inactivated gas such as argon or nitrogen gas substituted for oxygen in a reaction vessel, in the presence of an antioxidant, or under light-shieldedconditions, if necessary.

Reaction mixtures which contain the artepillin C and derivatives thereof of the present invention are obtainable by the above steps, and the optional decomposition reaction such as hydrolysis of ester bond or alcoholysis can be proceeded in a usual manner. The obtained reaction mixtures can be used intact or used after purified by the following conventional methods generally used for purifying their related compounds; dissolution, separation, extraction, decantation, filtration, concentration, thin-layer chromatography, column chromatography, high-performance liquid chromatography, distillation, sublimation, and crystallization. If necessary, two or more of these methods can be used in combination. Depending on reaction conditions and purification methods, in the above process for producing artepillin C and derivatives thereof of the present invention, the artepillin C and derivatives thereof can be produced in a yield of usually 15% or more, preferably 30% or more, and more preferably at least about 50% in a molar ratio of the artepillin C and derivatives thereof to the material phenol derivatives.

In many fields where one or two physiological activities of the produced artepillin C and derivatives thereof, for example, antibacterial, antioxidation, anti-inflammatory, anti-tumor, apoptosis-regulatory, immunoenhancement, and anti-lipidperoxidation activities can be used, the artepillin C and derivatives thereof can be advantageously used alone as a health food and supplement for biological activity of natural propolis extract, or used in the form of a composition including other ingredients. The present invention provides artepillin C and derivatives thereof which are produced by the process and compositions including the artepillin C and derivatives. Other ingredients, which can be incorporated in the composition of the present invention mean ingredients which are orally, percutaneously, or externally administrable to living bodies, such as alcohols, water, starches, proteins, amino acids, fibrous materials, saccharides, lipids, fatty acids, vitamins, minerals, flavoring agents, coloring agents, sweeteners, seasonings, spices, antiseptics, emulsifiers, and detergents. Examples of fields to utilize the composition of the present invention are fields such as food products, beverages, health foods, cosmetics, pharmaceuticals, household articles, agriculture, forestry, and fishery. Examples of the useful forms of food products are beverages such as juices, mineral drinks, and sports drinks; health foods in the forms of a tablet, capsule, and paste, etc; confectioneries such as candies, jellies, semi-hard jellies, caramels, chewing gums, and cakes, etc; ice sweet stuffs such as ice creams, bars of sherbet, etc; and seasonings such as soy sauce, sauce, mayonnaise, and dressing, etc. Examples of the useful forms in cosmetics are lotions, milky lotions, creams, basic cosmetics, hair cosmetics, cleaning cosmetics, aromatic cosmetics, oral cosmetics, and bathing cosmetics. Examples of the useful forms in pharmaceuticals are extracts, capsules, granules, pills, eye ointments, emulsions, plasters, powders, tablets, syrups, eye lotions, troches, ointments, and poultices, etc. Examples of useful forms in household articles are antibacterial- and germicidal goods, washing goods and cleaning goods. Examples of useful forms in agriculture, forestry and fishery are feeds for domestic animals, baits for fishes, insecticides, insect powders, and herbicides.

The preferred embodiments according to the resent invention are described hereinafter with reference to the following Examples:

<u>Example 1</u>

<u>Phenol derivative</u>

2.2 g (10 mmol) of 4-iodophenol was dissolved in 24 ml (60 mmol) of a 10% (w/v) aqueous sodium hydroxide solution. 3.72 g (25 mmol) of 1-bromo-3-methyl-2-butene was dropped in the solution under stirring and ice-cooling conditions, and the mixture was reacted for five hours at ambient temperature under stirring conditions. Apart of the reaction mixture was subjected to thin-layer chromatography using "Silicagel $60F_{254}$" commercialized by Merck & Co., Inc. as a thin-layer, and a mixture ofn-hexane/ethyl acetate (10:1 by volume) as a developing solvent. After the development, components in the reaction mixture were detected by irradiating ultraviolet rays with a wavelength of 254 nm or by coloring reaction with iodine. A remarkable spot, seemed a reaction product, was detected in a position of Rf of about 0.5.

The above reaction mixture was adjusted to acidic condition by adding 2 N aqueous acetic acid solution under ice-cooling conditions, followed by extracting with diethyl ether three times. The obtained extract was pooled, washed with water, dried over magnesium sulfuric anhydride, followed by removing the solvent under a reduced pressure to obtain 3.46 g of a reaction product. All of reaction product was subjected to silicagel column chromatography using n-hexane/diethyl ether (20:1 by volume) as a moving phase and fractionated. A part of each fraction was subjected to thin-layer chromatography as mentioned above, fractions that include ingredients with Rf of about 0.5 were pooled. The obtained mixture was removed solvents under reduced pressure to obtain 762 mg of a yellow-colored oily product.

According to a conventional manner, the obtained yellow-colored oily product was subjected to $^1$H-nuclear magnetic resonance spectroscopy, infra-red absorption spectroscopy, and ultraviolet absorption spectroscopy. The yellow-colored oily product was identified as 4-iodo-2,6-bis(3-methyl-2-butenyl) phenol based on the above analytical results and the chemical structures of reaction materials used in this Example. In this Example, the molar yield of the phenol derivative for 4-iodophenol as a reaction material was 20% or more. In reaction steps of this Example, the kinds and volume of by-products were relatively-few and low, and the phenol derivative was efficiently prepared by the method of this Example.

The analytical results of the above yellow-colored oily product were as follows. Upon measurement of $^1$H-nuclear magnetic resonance spectroscopy (400 MHz) in chloroform deuteride, the product showed a chemical shift δ (ppm, TMS) at peaks at 7.26 (2H, s, Ar-), 5.26 (2H, t, J=6.83Hz, $-CH=CMe_2$), 3.27 (4H, d,

J=6.83Hz, $ArCH_2$-), 1.77 (6H, s, $CH_3$-) , and 1.75 (6H, s, $CH_3$-). "Ar" and "Me" mean an aryl and methyl groups, respectively. Upon measurement of infra-red absorption spectroscopy by pressure tablet method using powdery potassium bromide, the product showed distinctive absorption peaks at 3444 $cm^{-1}$ (broad) , 2954 $cm^{-1}$, 2914 $cm^{-1}$, and 1601 $cm^{-1}$. Upon measurement of ultraviolet absorption spectroscopy, the product showed absorption peaks at 206 nm and 236 nm, and the molar absorption coefficients ε at the peaks were respectively 27200 (206 nm) and 11160 (236 nm).

Chemical Formula 1

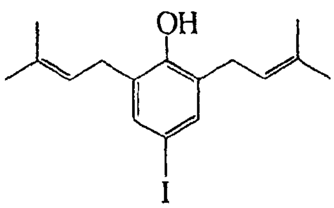

Example 2

Phenol derivative

After the air in a reaction vessel was replaced with a sufficient amount of argon gas, and the reaction of 4-iodophenol with 1-bromo-3-methyl-2-butene as disclosed in Example 1 was carried out under light-shielded conditions. According to the method in Example 1, the reaction mixture was extracted with an organic solvent, and the obtained extract was washed, dried, and distilled to remove solvent under reduced pressure to obtain a reaction product. All of the reaction product was subjected to silica-gel column chromatography to obtain a phenol derivative represented by Chemical Formula 1. The obtained compound was subjected to $^1$H-nuclear magnetic resonance spectroscopy, infra-red absorption spectroscopy, and ultraviolet absorption spectroscopy. It was confirmed that these analytical results were identical to the results of Example 1. In this Example, the molar yield of the phenol derivative to 4-iodophenol as the reaction material was about 40%. In the reaction steps of this Example, the kinds and volume of by-products were relatively few and low, and the phenol derivative was efficiently prepared by the method of this Example.

Example 3

Phenol derivative

The phenol derivative of the present invention represented by Chemical Formula 3, which was identified as 4-bromo-2,6-bis(3-methyl-2-butenyl) phenol, was obtained similarly as in Example 1 except for replacing 4-iodophenol with 1.73 g (10 mmol) of 4-bromophenol. Upon measurement of ultraviolet absorption spectroscopy in a conventional manner, the phenol derivative showed absorption peaks at 204 nm and 230 nm and the molar absorption coefficients ε at the peaks were 15494 (204 nm) and 4068 (230 nm). Upon measurement of infra-red absorption spectroscopy by pressure tablet method using powdery potassium bromide in a conventional manner, the phenol derivative showed distinctive absorption peaks at 3464 $cm^{-1}$ (broad), 2927 $cm^{-1}$, 2841 $cm^{-1}$, and 1729 $cm^{-1}$. The molar yield of the phenol derivative to 4-bromophenol as a material was about 20% or more. In reaction steps of this Example, the kinds and volume of by-products were relatively few and low, and the phenol derivative was efficiently prepared by the method of this Example.

Chemical Formula 3

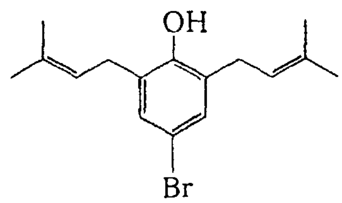

Example 4

Phenol derivative

After the air in a reaction vessel was replaced with a sufficient amount of argon gas, and the reaction of 4-bromophenol with 1-bromo-3-methyl-2-butene as disclosed in Example 1 was carried out under light-shielded conditions. According to the method in Example 1, the reaction mixture was subjected to silica-gel column chromatography to obtain a phenol derivative represented by Chemical Formula 3. The obtained phenol derivative was subjected to $^1$H-nuclear magnetic resonance spectroscopy, infra-red absorption spectroscopy, and ultraviolet absorption spectroscopy. It was confirmed that these analytical results were identical to the results of Example 3. A molar yield of the phenol derivative to 4-iodophenol as the reaction material was about 40%. In the reaction steps of this Example, the kinds and volume of by-products were relatively few and low, and the phenol derivative was efficiently prepared by the method of this Example.

Example 5

Preparation of artepillin C derivative

1.67 g (4.69 mmol) of the phenol derivative represented by Chemical Formula 1, which was obtained by the method in Example 1, and 2.00 g (23.3 mmol) of methyl acrylate were reacted in seven milliliters of an anhydrous toluene solution containing 0.944 g (9.33 mmol) of triethylamine, 14.3 mg (0.470 mmol) of tri-o-toluic phosphine, and 52 mg (0.232 mmol) of palladium acetate (II) at 100 °C for 20 hours under refluxing conditions. The reaction mixture was cooled, and a part of the reaction mixture was subjected to thin-layer chromatography. After a development, components of the reaction mixture were detected by irradiating ultraviolet rays with a wavelength of 254 nm. A remarkable spot seemed a reaction product was detected in the position of Rf of about 0.2.

The cooled reaction mixture was diluted by appropriate volume of a mixture of ethyl acetate/diethyl ether (1:1 by volume) and filtrated by passing through a celite. The obtained filtrate was washed with a saturated ammonium chloride solution. The washed aqueous phase was extracted with appropriate volume of a mixture of ethyl acetate/diethyl ether (1:1 by volume). This extract was mixed with the filtrate, dried over magnesium sulfuric anhydride, followed by removing solvent under reduced pressure to obtain 1.83 g of a reaction product. According to a conventional manner, all of the reaction product was subjected to silica-gel column chromatography using n-hexane/ethyl acetate (30:1 by volume) as a moving phase and fractionated. A part of each fraction was subjected to thin-layer chromatography as mentioned above, fractions which include ingredients with Rf of about 0.2 were pooled. The obtained mixture was distilled to remove solvents under reduced pressure to obtain 521 mg of a brown-colored oily product.

According to a conventional manner, the obtained brown-colored oily product was subjected to $^1H$-nuclear magnetic resonance spectroscopy (400 MHz) in chloroform deuteride, revealing that the material showed a chemical shift δ (ppm, TMS) at peaks at 7.60 (1H, d, J=16.0Hz, ArCH=), 7.17 (2H, s, Ar-), 6.28 (1H, d, J=16.0Hz, MeOCOCH=), 5.66 (1H, s, HO-), 5.31 (2H, t, J=6.83Hz, $Me_2C$=CH-), 3.79 (3H, s, $CH_3O$-), 3.34 (4H, d, J=6.83Hz, $ArCH_2$-), 1.79 (6H, s, $CH_3$-), and 1.77 (6H, s, $CH_3$-). "Ar" and "Me" mean an aryl and methyl groups, respectively. Upon measurement of infra-red absorption spectroscopy by pressure tablet method using powdery potassium bromide in a conventional manner, the material showed distinctive absorption peaks at 3445 $cm^{-1}$ (broad), 2973 $cm^{-1}$, 2915 $cm^{-1}$, 1718 $cm^{-1}$, 1696 $cm^{-1}$, 1633 $cm^{-1}$, and 1599 $cm^{-1}$. The brown-colored oily product was identified with 3- [ 4-hydroxy-3,5-bis(3-methyl-2-butenyl) phenol ] -methyl 2-propenoate by the above analytical results and chemical structures of reaction materials used in this Example. In this Example, the molar yield of the artepillin C and derivatives thereof to the phenol derivative represented by Chemical Formula 1 as the reaction material was about 35%. In the reaction steps of this Example, the kinds and volume of by-products were relatively few and low, and the phenol derivative was efficiently prepared by the method of this Example.

Chemical Formula 4

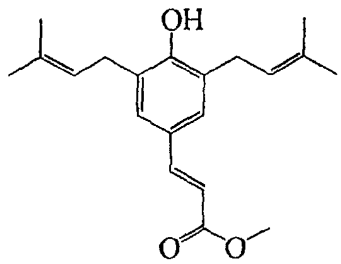

Example 6

Preparation of artepillin C derivative

The phenol derivative represented by Chemical Formula 1 in Example 5 and methyl acrylate were reacted under continuous supplying of argon gas into a reaction mixture and under light-shielded conditions. According to the method in Example 5, a reaction mixture was filtrated, and the filtrate was washed, dried, and distilled to remove solvents under reduced pressure to obtain a reaction product. The reaction product was subjected to silica-gel column chromatography to obtain artepillin C and derivatives thereof represented by Chemical Formula 4. The obtained compound was subjected to $^1H$-nuclear magnetic resonance spectroscopy and infra-red absorption spectroscopy. It was confirmed that these analytical results were identical to results of Example 5. In this Example, the molar yield of a compound represented by Chemical Formula 4 obtained in this Example to the phenol derivative as the reaction material was about 50%. In reaction steps of this Example, the kinds and volume of by-products were relatively few and low, and the artepillin C and derivatives thereof was efficiently prepared by the method of this Example.

Example 7

Preparation of artepillin C derivative

Two hundred and seventy-four milligrams of the compound represented by Chemical Formula 4 obtained by the method in Example 5 was reacted in a mixture of 15 ml of a 10% (w/v) aqueous potassium hydroxide solution and 15 ml of methanol at 100 °C for one hour under refluxing conditions. The reaction mixture was cooled, and a part of the reaction mixture was subjected to thin-layer chromatography using "Silica Gel $60F_{254}$" commercialized by Merck & Co., Inc. as thin-layer and a mixture of chloroform/methanol (20:1 by volume) as a developing solvent. After a development, components of the mixture were detected by irradiating of ultraviolet rays with a wavelength of 254 nm or by

coloring reaction with iodine. A remarkable spot seemed to be a reaction product was detected in the position of Rf of about 0.5.

The cooled reaction mixture was adjusted to acidic condition by 1 N hydrochloric acid and extracted with ethyl acetate two times. The extract was mixed and washed with a saturated ammonium chloride and further washed with a saturated saline solution. The washed extract was dried over magnesium sulfuric anhydride, and distilled to remove solvents under reduced pressure to obtain 306 mg of a reaction product. All of the reaction product was subjected to silica-gel column chromatography using chloroform/methanol (20:1 by volume) as a moving phase and fractionated. A part of each fraction was subjected to thin-layer chromatography as mentioned above, fractions which include ingredients with Rf of about 0.5 were pooled. The obtained mixture was distilled to remove solvents under reduced pressure to obtain 224 mg of a light-yellow-colored solid. This light-yellow-colored solid was finely crushed and washed in n-hexane to obtain 181 mg of a white-colored crystal.

According to a conventional manner, the obtained white-colored crystal was subjected to $^1$H-nuclear magnetic resonance spectroscopy, infra-red absorption spectroscopy, and ultraviolet absorption spectroscopy. The white-colored solid was identified with 3-[4-hydroxy-3,5-bis(3-methyl-2-butenyl) phenyl] -2-propenoic acid represented by Chemical Formula 2 of artepillin C, by these analytical results and chemical structures of reaction materials used in this Example. In this Example, the molar yield of artepillin to the compound represented by Chemical Formula 4 as the reaction material was about 70%.

The analytical results of above white-colored crystal were showed as follows. Melting point was 98-99 °C. Upon measurement of $^1$H-nuclear magnetic resonance spectroscopy (400 MHz ) in chloroform deuteride, the material showed a chemical shift δ (ppm, TMS) at peaks at 7.69 (1H, d, J=15.6Hz, ArCH=), 7.20 (2H, s, Ar-), 6.29 (1H, d, J=15.6Hz, MeOCOCH=), 5.31 (2H, t, J=6.84 Hz, $Me_2C$=CH-), 3.35 (4H, d, J=6.84Hz, $ArCH_2$-), 1.79 (6H, s, $CH_3$-), and 1.78 (6H, s, $CH_3$-). "Ar" and "Me" mean an aryl and methyl groups, respectively. Upon measurement of infra-red absorption spectroscopy by pressure tablet method using powdery potassium bromide, the material showed distinctive absorption peaks at 3420 $cm^{-1}$ (broad), 2976 $cm^{-1}$, 2926 $cm^{-1}$, 1685 $cm^{-1}$, 1629 $cm^{-1}$, and 1598 $cm^{-1}$. Upon measurement of ultraviolet absorption spectroscopy, the material showed absorption peaks at 220 nm, 237 nm and 314 nm and molar absorption coefficients ε at the peaks were each 11328 (220 nm), 13053 (237 nm), and 17329 (314 nm).

Chemical Formula 2

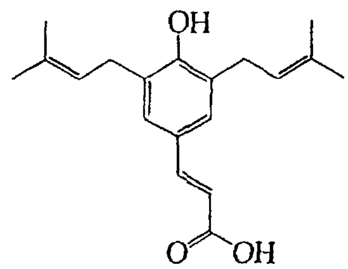

Example 8

Preparation of artepillin C derivative

The compound represented by Chemical Formula 4 in Example 7 as a starting material was reacted under light-shielded conditions while continuously supplying argon gas into a reaction mixture. According to the method in Example 7, the reaction mixture was adjusted to acidic conditions and extracted with organic solvents. The extract was washed, dried, and distilled to remove solvents under reduced pressure to obtain a reaction product. The reaction product was subjected to silica-gel column chromatography to obtain artepillin C represented by Chemical Formula 2. The obtained compound was analyzed according to the method in Example 7. It was confirmed that these analytical results were identical to results of Example 7. The molar yield of artepillin C obtained in this Example to the compound represented by Chemical Formula 4 as the reaction material was about 80%.

Example 9

Preparation of artepillin C derivative

The artepillin C derivative represented by Chemical Formula 4 was obtained similarly as in Example 5 except for replacing phenol derivative represented by Chemical Formula 1 with phenol derivative represented by Chemical Formula 3 obtained in Example 3. The obtained artepillin C and derivatives thereof were operated according to Example 7 to obtain artepillin C represented by Chemical Formula 2. The obtained compound was analyzed according to the method in Example 7. It was confirmed that these analytical results were identical to results of Example 7. The molar yield of artepillin C obtained in this Example to the phenol derivative represented by Chemical Formula 3 was about 20%.

Example 10

Preparation of artepillin C derivative

The phenol derivative represented by Chemical Formula 1 obtained by the method in Example 1 and 5-time volumes, in a molar ratio, of acrylic acid to the phenol derivative were reacted in a mixture of dimethylformamide/water (2:8 by volume) in the presence of triethylamine tri-o-toluic phosphine and palladium acetate (II) at 100 °C for 20 hours under refluxing conditions. The reaction mixture was extracted with organic solvents. According to a conventional manner, the extract was subjected to silica-gel column chromatography using a mixture of chloroform/methanol (20:1 by volume) as a developing solvent to obtain artepillin C represented by Chemical Formula 2 as a light-yellow-colored solid. This light-colored solid was washed in n-hexane to obtain a white-colored crystal of artepillin C.

In reaction steps of this Example, the kinds and volume of by-products were relatively few and low, and artepillin C was efficiently prepared by the method of this Example.

Example 11

Preparation of artepillin C derivative

The phenol derivative represented by Chemical Formula 1 obtained by the method in Example 1 and 1.2-time volumes, in a molar ratio, of acetyl chloride to the phenol derivative were reacted in methylene chloride in the presence of triethylamine and dimethylaminopyridine according to normal acetylate reaction to obtain an acetylated compound of phenol derivative represented by Chemical Formula 1.

To the obtained acetylated compound and five times volume of methyl acrylate as the compound in a molar ratio were reacted according to Example 2. The reaction mixture was extracted with organic solvents. According to a conventional manner, the extract was washed, dried and distilled to remove solvents under reduced pressure to obtain a reaction product. The reaction product was subjected to silica-gel column chromatography using a mixture of n-hexane/ethyl acetate as a moving phase to obtain a compound represented by Chemical Formula 5 of artepillin C and derivatives thereof.

Chemical Formula 5

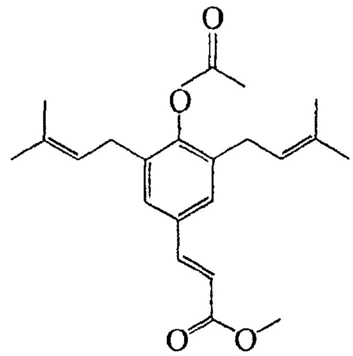

To the resulting compound represented by Chemical Formula 5 was added adequate amounts of a 10% potassium hydroxide solution and methanol, and the mixture was reacted at 100 °C for one hour under refluxing. According to a conventional manner, the reaction mixture was extracted, washed, dried and distilled to remove solvents. The obtained reaction product was subjected to silica-gel column chromatography using a mixture of chloroform/methanol as a moving phase to obtain artepillin C represented by Chemical Formula 2 as a light-yellow-colored solid. The light-yellow-colored solid was washed with n-hexane to obtain a white-colored crystal of artepillin C.

In a reaction of an acetylated compound and methyl acrylate of this Example, the kinds and yields of by-products were relatively few and low, and artepillin C was efficiently prepared by the method of this Example.

Example 12

Liquid composition

Four parts by weight of artepillin C derivative, obtained by the method in Example 7, as dissolved to 96 parts by weight of 80% (v/v) ethanol to obtain a liquid composition. The composition can be advantageously used as a household article such as an antibacterial- and germicidal good alone and as an addition which add a biological action of artepillin C in food products, cosmetics and pharmaceuticals.

Example 13

Gummy Candy

One hundred and fifty parts by weight of a hydrogenated maltose syrup (registered trade mark of "MABIT" commercialized by Hayashibara Shoji, Inc., Okayama, Japan) was heated and concentrated to give a moisture content of about 15% (w/w) under reduced pressure. According to a conventional manner, the concentrated solution was added a solution dissolved 13 parts by weight of gelatin to 18 parts by weight of water,

four parts by weight of the liquid composition in Example 12, two parts by weight of citric acid, appropriate volume of a coloring agent and flavor, mixed, formed and packed to obtained a gummy candy. The gummy candy, which has a good texture and flavor and scarcely causes bad teeth, is also useful as a health food to maintain/promote health.

Example 14

Chewing Gum

Three parts by weight of a gum base was melted to be softened by heating and admixed with four parts by weight of sucrose, three parts by weight of maltose powder, and further mixed with 0.04 part by weight of the liquid composition in Example 12 and a coloring agent. According to a conventional manner, the resulting mixture was kneaded by a roll, formed, and packed to obtain a chewing gum. The chewing gum, which has a good texture and flavor, and scarcely causes dental caries, can be also useful as a health food to maintain/promote health.

Example 15

Milky Lotion

According to a conventional manner, 0.5 part by weight of polyoxyethylene behenyl ether, one part by weight of tetraoleic acid polyoxyethylene sorbitol, one part by weight of lipophilic glycerol monosterate, 0.5 part by weight of pyruvic acid, 0.3 part by weight of behenyl alcohol, 0.3 part by weight of maltitol, one part by weight of avocado oil, one part by weight of a liquid composition in Example 12, appropriate volume of vitamin E, and antiseptic were melted by heating. The obtained mixture was added one part by weight of L-sodium lactate, seven parts by weight of 1,3-buthylene glycol, 0.1 part by weight of carboxyvinyl polymer, and 86.3 parts by weight of refined water, and emulsified by using a homogenizer. The obtained emulsion was admixed a flavor by shaking to obtain a milky lotion. The milky lotion, which spreads well and was not sticky, is also useful as cosmetics to maintain/promote dermal health.

Example 16

Ointment

According to a conventional manner, one part by weight of sodium acetate trihydrate and four parts by weight of DL-potassium lactate were uniformly mixed to ten parts by weight of glycerol. The obtained mixture was added 0.5 part by weight of mint oil, 49 parts by weight of petrolatum, 10 parts by weight of Japan wax, 10 parts by weight of lanoline, 14.5 parts by weight of sesame oil, and one part by weight of the liquid composition in Example 12, and uniformly mixed to obtain an ointment. The ointment, which exerts a good osmolar and extensibility to skin, was also useful as pharmaceuticals to maintain/promote dermal health.

As described above, the present invention is made based on the establishment of novel phenol derivatives, which are useful as a material of artepillin C and derivatives thereof, and their preparations. As the process for producing artepillin C and derivatives thereof by organic synthesis of the present invention is very efficiently proceeding under moderate conditions and by-products are relatively-few and low, it is useful to produce artepillin C and derivatives thereof, more particularly, to industrially produce a purified sample of artepillin C and derivatives thereof which can be applied to living bodies. The compounds, which were produced by the process for producing artepillin C and derivatives thereof of the present invention, are advantageously useful in many fields such as food products, beverages, health foods, cosmetics, pharmaceuticals, household articles, agriculture, forestry, and fishery which physiological activities of artepillin C and derivatives thereof can be utilized.

The present invention with such outstanding effects and functions is a significant invention that will greatly contribute to this art.

**Claims**

1. A phenol derivative represented by Formula 1 :

Formula 1

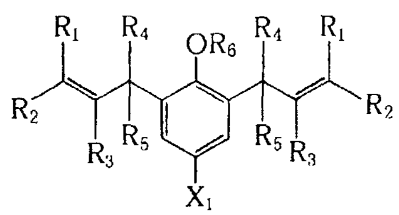

wherein in Formula 1,
$R_1$, $R_2$, $R_3$, $R_4$, and $R_5$ each independently represent a hydrogen atom, an aliphatic hydrocarbon group, alicyclic hydrocarbon group, or aromatic hydrocarbon group, and these hydrocarbon groups optionally have a substituent;
$R_6$ represents a hydrogen atom or an acyl group; and
$X_1$ represents a substituent selected from halogens, nitro, nitroso, cyano, mercapto, sulfonic acid, and sulfonyl groups.

2. The phenol derivative of claim 1, wherein $R_6$ in Formula 1 is a hydrogen atom or an acetyl group, and

$X_1$ in Formula 1 is a halogen.

**3.** The phenol derivative of claim 1 or 2, which is represented by Chemical Formula 1:

Chemical Formula 1

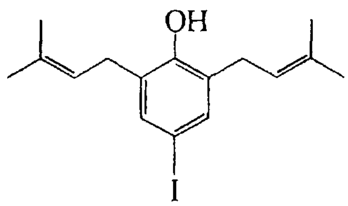

**4.** A process for producing the phenol derivative of any one of claims 1 to 3, comprising a step of reacting a compound represented by Formula 2 having $X_1$, which corresponds to Formula 1, with a compound represented by Formula 3 having $R_1$ to $R_5$ which correspond to Formula 1 :

Formula 2

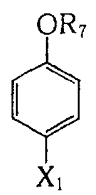

wherein in Formula 2, $R_7$ represents a hydrogen atom or an acyl group,

Formula 3

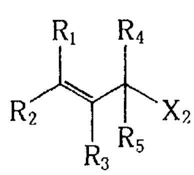

wherein in Formula 3, $X_2$ represents a halogen.

**5.** The process of claim 4, wherein the compound represented by Formula 3 is used in the step of reacting their compounds in an amount of exceeding one fold of the compound represented by Formula 2 in a molar ratio.

**6.** The process of claim 4 or 5, wherein the step of reacting the compounds represented by Formulae 2 and 3 is effected under alkaline conditions.

**7.** The process of any one of claims 4 to 6, wherein the compound represented by Formula 2 is 4-iodophenol, and the compound represented by Formula 3 is 1-bromo-3-methyl-2-butene.

**8.** A food product or cosmetic comprising the phenol derivative of any one of claims 1 to 3.

**9.** A process of producing 3- [4-hydroxy-3,5-bis-(3-methyl-2-butenyl)phenyl] -2-propenic acid and a derivative thereof represented by Formula 5, comprising a step of reacting the phenol derivative of any one of claims 1 to 3 with acrylic acid or an acrylate represented by Formula 4 :

Formula 4

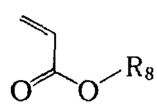

wherein in formula 4, $R_8$ represents an aliphatic, alicyclic, or aromatic hydrocarbon group, and these hydrocarbon group optionally have a substituent,

Formula 5

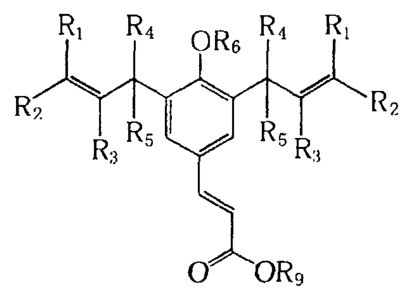

wherein in Formula 5, $R_1$ to $R_6$ are defined similarly as in Formula 1, and $R_9$ represents a hydrogen atom, an aliphatic hydrocarbon group, alicyclic hydrocarbon group, or aromatic hydrocarbon group, and these hydrocarbon groups optionally have a substituent.

**10.** The process of claim 9, further comprising a step of hydrolysis or alcoholysis.

**11.** The process of claim 9 or 10, wherein the compound represented by Formula 5 is 3- [4-hydroxy-3,5-bis-(3-methyl-2-butenyl)phenyl] -2-propenoic acid represented by Chemical Formula 2.

Chemical Formula 2

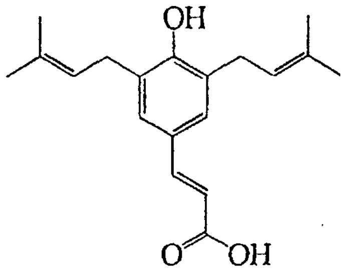

**12.** A composition comprising 3- [4-hydroxy-3,5-bis-(3-methyl-2-butenyl)phenyl] -2-propenic acid and/or a derivative thereof represented by Formula 5, produced by the method of any one of claims 9 to 11.

**13.** The composition of claim 12, which is in the form of a food product, cosmetic or pharmaceutical.

## INTERNATIONAL SEARCH REPORT

International application No.
PCT/JP01/02482

**A. CLASSIFICATION OF SUBJECT MATTER**

Int.Cl$^7$ C07C39/373, C07C37/18, C07C69/73, C07C67/343, A61K31/055, A61K31/216, A61K31/192, A61K31/235, A61K7/00, A23G3/30, A23G3/00 // C07C69/732, C07C59/54

According to International Patent Classification (IPC) or to both national classification and IPC

**B. FIELDS SEARCHED**

Minimum documentation searched (classification system followed by classification symbols)

Int.Cl$^7$ C07C39/373, C07C37/18, C07C69/73, C07C67/343, A61K31/055, A61K31/216, A61K31/192, A61K31/235, A61K7/00, A23G3/30, A23G3/00 // C07C69/732, C07C59/54

Documentation searched other than minimum documentation to the extent that such documents are included in the fields searched

Electronic data base consulted during the international search (name of data base and, where practicable, search terms used)

CA (STN)

**C. DOCUMENTS CONSIDERED TO BE RELEVANT**

| Category* | Citation of document, with indication, where appropriate, of the relevant passages | Relevant to claim No. |
|---|---|---|
| X | JP, 54-117486, A (Sankyo Company, Limited), 12 September, 1979 (12.09.79), 4-chloro-2,6-diallyl phenol(RN:73267-76-2)which is the raw material has also substantially shown, as 2-(4-chloro-2,6-diallyl phenoxy)-5-bromo pyrimidine has been described (in Japanese) (Family: none) | 1,2 |
| X | JP, 4-288031, A (Fuji Photo Film Co., Ltd.), 13 October, 1992 (13.10.92), X is -SO2- chemical compound in general equation I (in Japanese) (Family: none) | 1 |
| A<br>X | JP, 61-47433, A (Res. Inst. for Prod. Dev.), 07 March, 1986 (07.03.86), page 2, upper column, synthesis chart (Family: none) | 1-11<br>12,13 |
| A<br>X | JP, 59-118736, A (Res. Inst. for Prod. Dev.), 09 July, 1984 (09.07.84), page 2, upper left column, synthesis chart (Family: none) | 1-11<br>12,13 |

☒ Further documents are listed in the continuation of Box C.  ☐ See patent family annex.

* Special categories of cited documents:
- "A" document defining the general state of the art which is not considered to be of particular relevance
- "E" earlier document but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| 05 June, 2001 (05.06.01) | 26 June, 2001 (26.06.01) |
| Name and mailing address of the ISA/<br>Japanese Patent Office | Authorized officer |
| Facsimile No. | Telephone No. |

Form PCT/ISA/210 (second sheet) (July 1992)

**INTERNATIONAL SEARCH REPORT**

International application No.
PCT/JP01/02482

C (Continuation). DOCUMENTS CONSIDERED TO BE RELEVANT

| Category* | Citation of document, with indication, where appropriate, of the relevant passages | Relevant to claim No. |
|---|---|---|
| A<br>X | JP, 60-163841, A (Res. Inst. for Prod. Dev.),<br>26 August, 1985 (26.08.85),<br>page 2, upper right column to page 2, lower left column, synthesis chart (Family: none) | 1-11<br>12,13 |
| A<br>X | JP, 6-256177, A (Hayashibara Biochemical Lab. Inc.),<br>13 September, 1994 (13.09.94),<br>Claims (Family: none) | 1-11<br>12,13 |
| A<br>X | GB, 2302809, A (Kabushiki Kaisha Hayashibara Seibutsu Kagaku Kenkyujyo),<br>05 February, 1997 (05.02.97),<br>Claims<br>& US, 5710179, A1 & JP, 9-71528, A | 1-11<br>12,13 |
| X | VDOVTSOVA, E.A. et.al, "Alkylation of aromatic compounds by diene hydrocarbons and their hydrochlorides. XXV. α,γ-Dimethylallylation of p-chlorophenol and p-chloroanisole by 4-chloro-2-pentene and piperylene", Deposited Publ. (1972), VINITI 4340-72 (CA 85:46313)<br>4-chloro-2,6-bis (1-methyl-2-butenyl) phenol compounds (RN:59836-33-8) | 1,2 |
| X | Tadashi SUEHIRO et. al, "Über die Darstellung einiger 5-Oxo-2-methyl-4, 4-dialkyl-3-äthoxycarbonyl-4,5-dihydroindol und deren Reduktionen",<br>BULLETIN OF THE CHEMICAL SOCIETY OF JAPAN, Vol.40 pp2925-2930 (1967) compounds Ⅷ (RN:18377-46-3) | 1 |
| X | Chemical Abstracts, Vol.55 (1961), 21021d, "polyethers. Ⅶ. poly(2,6-disubstituted-1,4-phenylene oxides",<br>2,6-diallyl-4-bromophenol compounds (RN:17362-23-1) | 1 |

Form PCT/ISA/210 (continuation of second sheet) (July 1992)

## INTERNATIONAL SEARCH REPORT

International application No.
PCT/JP01/02482

**Box I Observations where certain claims were found unsearchable (Continuation of item 1 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

Compounds represented by the general formula 5 in claim have been publicly known at the time of the international application, so that the special technical features of this application are considered as residing in compounds of the general formula 1. However, the inventions of claims 12 and 13 have special technical features different from the above ones (i.e., the technical features of the inventions of claims 12 and 13 reside in compositions containing compounds of the general formula 5). Therefore, there is no technical relationship among the inventions involving one or more of the same or corresponding technical features. Such being the case, the inventions are not considered as relating to a group of inventions so linked as to form a single general inventive concept.

1. ☐ As all required additional search fees were timely paid by the applicant, this international search report covers all searchable claims.

2. ☒ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.
☐ No protest accompanied the payment of additional search fees.

Form PCT/ISA/210 (continuation of first sheet (1)) (July 1992)